# EUROPEAN PATENT APPLICATION

(11) **EP 4 585 693 A1**
(43) Date of publication of application: **16.07.2025**
(21) Application number: 23874471.8
(22) Date of filing: 24.05.2023
(51) Int. Cl.: C12P 9/00, A23D 7/01, C11B 11/00, C11C 1/06

(54) **METHOD FOR PRODUCING LYSOPHOSPHOLIPID-CONTAINING COMPOSITION AND METHOD FOR PRODUCING OIL-IN-WATER EMULSION COMPOSITION USING SAME**

(30) Priority: 06.10.2022 JP 2022161328
(71) Applicant: Kewpie Corporation, Tokyo 150-0002 (JP)
(72) Inventor: SUSAKI Kenta, Chofu-shi, Tokyo 182-0002 (JP); YAMAMOTO Hiroyoshi, Chofu-shi, Tokyo 182-0002 (JP); KOBAYASHI Hideaki, Chofu-shi, Tokyo 182-0002 (JP); HOSHINA Ryosuke, Chofu-shi, Tokyo 182-0002 (JP); ISHIKAWA Takuya, Chofu-shi, Tokyo 182-0002 (JP)
(74) Representative: Winter, Brandl - Partnerschaft mbB
(86) International application number: PCT/JP2023/019359
(87) International publication number: WO 2024/075335

(57) **Abstract**

The present invention relates to a method for producing a lysophospholipid-containing composition in which a phospholipid is decomposed by phospholipase A, the method includes a mixing step, a phospholipid enzymolysis step, and an enzyme inactivation step, a value calculated by a L2 value - a L1 value is positive in the lysophospholipid-containing composition.
the L1 value: a brightness value of the composition,
the L2 value: a brightness value of an oil-in-water emulsion obtained by dispersing the composition in 100 times by mass of pure water

In the mixing step, a total amount of a phospholipid, a fat and oil, a polyhydric alcohol, pure water, and phospholipase A used is 90% by mass or more with respect to a total amount of the raw materials and the phospholipase A, a content of the polyhydric alcohol in the raw materials is 0.4 to 3.0 times by mass with respect to a content of the pure water, a content of the phospholipid in the raw materials is 0.07 to 0.5 times by mass with respect to a total content of the phospholipid and the fat and oil, and a total amount of the polyhydric alcohol and the pure water in the raw materials is 0.25 to 4.0 times by mass with respect to the total content of the phospholipid and the fat and oil.

## Description

### Technical Field

The present invention relates to a method for producing a lysophospholipid-containing composition, in which a phospholipid is decomposed into a lysophospholipid by a novel method in which the enzymolysis of the phospholipid by phospholipase A proceeds without using an organic solvent accompanied by removal, and the lysophospholipid-containing composition to be obtained has self-emulsifying properties and is easily used for various applications, and a method for producing an oil-in-water emulsion composition using the lysophospholipid-containing composition.

### Background Art

As a method for decomposing a phospholipid by phospholipase A, for example, a method for dissolving a phospholipid in an organic solvent and bringing the phospholipid into contact with phospholipase A while mixing the organic solvent phase with a dispersion liquid of the phospholipase A (Non-Patent Literature 1), a method for preparing an oil-in-water emulsion using a phospholipid as an emulsifier, and bringing the phospholipase A dispersed in a water phase into contact with the phospholipid present at an interface between the oil phase and the water phase (Non-Patent Literature 2), and the like are known.

However, in the method described in Non-Patent Literature 1, the enzyme reaction proceeds easily by using an organic solvent, but the resultant product cannot be used for various applications, for example, foods, pharmaceuticals, and the like, unless the organic solvent is removed. In particular, the manufacturing conditions using diethyl ether described in Non-Patent Literature 1 are not permitted for food applications due to safety. In addition, in the method described in Non-Patent Literature 2, the phospholipid can be enzymatically decomposed in a state of an oil-in-water emulsion without using an organic solvent, but there is a problem that the enzymolysis reaction rate is slower than that in a method using an organic solvent, and a large amount of energy is required to finally remove water. Any of these methods have some problems, and a new enzymolysis method in which these problems are solved has been required.

### Citation List

### Non Patent Literature

[Non-Patent Literature 1] Biochemical Experiment Course 3 "Chemistry of Lipids", edited by the Japanese Biochemical Society, pp. 264 to 265, published on November 25, 1974.
[Non-Patent Literature 2] D. M. Cabezas, R. Madoery, B. W. K. Diehl, M. C. Tomás, Emulsifying Properties of Different Modified Sunflower Lecithins, J. Am. Oil Chem. Soc. (2012) 89: pp. 355-361.

### Summary of Invention

### Technical Problem

An object of the present invention is to provide a method for producing a lysophospholipid-containing composition, in which a phospholipid is decomposed into a lysophospholipid by a novel method in which the enzymolysis of the phospholipid by phospholipase A proceeds without using an organic solvent accompanied by removal, and the lysophospholipid-containing composition to be obtained has self-emulsifying properties and is easily used for various applications, and a method for producing an oil-in-water emulsion composition using the lysophospholipid-containing composition.

### Solution to Problem

The present inventors have conducted intensive studies to achieve the above object.

As a result, it was found that, in a case of attempting a novel enzymolysis in which the phospholipid is subjected to enzymolysis by phospholipase A in a polyhydric alcohol-based mixture containing a phospholipid, a fat and oil, a polyhydric alcohol, and a small amount of pure water as raw materials, and the mixing ratio of the raw materials of the mixture is controlled such that the obtained composition has self-emulsifying properties, the enzymolysis of the phospholipid by phospholipase A proceeds without using an organic solvent accompanied by removal, and a lysophospholipid-containing composition having self-emulsifying properties and being easily used for various applications is obtained, thereby completing the present invention.

That is, present invention relates to
(1) a method for producing a lysophospholipid-containing composition, in which the lysophospholipid-containing composition obtained by decomposing a phospholipid with phospholipase A, the method including:
   a mixing step of uniformly mixing raw materials containing a phospholipid, a fat and oil, a polyhydric alcohol, and pure water, and phospholipase A to initiate enzymolysis of the phospholipid by the phospholipase A;
   a phospholipid enzymolysis step subjecting the phospholipid to the enzymolysis by the phospholipase A such that a decomposition ratio of the phospholipid in a mixture obtained in the mixing step is 20% by mass or more; and
   an enzyme inactivation step inactivating the phospholipase A in an enzymolysis product obtained in the phospholipid enzymolysis step,
   in which a value calculated by a L2 value - a L1 value is positive in the obtained lysophospholipid-containing composition,
   the L1 value: a brightness value of the lysophospholipid-containing composition,
   the L2 value: a brightness value of an oil-in-water emulsion obtained by dispersing the lysophospholipid-containing composition in 100 times by mass of pure water,
   a total amount of the phospholipid, the fat and oil, the polyhydric alcohol, the pure water, and the phospholipase A used in the mixing step is 90% by mass or more with respect to a total amount of the raw materials and the phospholipase A used,
   a content of the polyhydric alcohol in the raw materials is 0.4 to 3.0 times by mass with respect to a content of the pure water in the raw material,
   a content of the phospholipid in the raw materials is 0.07 to 0.5 times by mass with respect to a total content of the phospholipid and the fat and oil in the raw material, and
   a total content of the polyhydric alcohol and the pure water in the raw materials is 0.25 to 4.0 times by mass with respect to the total content of the phospholipid and the fat and oil in the raw material,
(2) the method for producing a lysophospholipid-containing composition according to (1), in which the value calculated by the L2 value - the L1 value is +5 or more,
(3) a method for producing an oil-in-water emulsion composition, including a water dispersion step of dispersing the lysophospholipid-containing composition obtained by the method for producing a lysophospholipid-containing composition according to (1) or (2) in pure water,
(4) a method for producing a mixed composition, including a mixing step of mixing one or two or more of a fat and oil, a lipid-soluble substance, a polyhydric alcohol, pure water, and a water-soluble substance with the lysophospholipid-containing composition obtained by the method for producing a lysophospholipid-containing composition according to (1) or (2), in which a value calculated by a L2 value - a L1 value is positive in the mixed composition obtained by the mixing step,
   the L1 value: a brightness value of the mixed composition,
   the L2 value: a brightness value of an oil-in-water emulsion obtained by dispersing the mixed composition in 100 times by mass of pure water, and
(5) a method for producing an oil-in-water emulsion composition, including a water dispersion step of dispersing the mixed composition obtained by the method for producing a mixed composition according to (4) in pure water.

### Advantageous Effects of Invention

According to the present invention, it is possible to provide a composition in which a phospholipid is decomposed into a lysophospholipid by a novel method in which the enzymolysis of the phospholipid by phospholipase A proceeds without using an organic solvent accompanied by removal, and the lysophospholipid-containing composition to be obtained has self-emulsifying properties and is easily used for various applications. Therefore, it is possible to provide a lysophospholipid-containing composition obtained by decomposing a phospholipid at a desired decomposition ratio, and the expansion of the use of the composition, in particular, the expansion of the use thereof into an oil-in-water emulsion composition is expected because of the characteristics of the composition.

### Description of Embodiments

Hereinafter, the present invention will be described in detail.

It is noted that in the present invention, unless otherwise specified, "%" means "% by mass", "times" means "times by mass", and "parts" means "parts by mass".

### <Features of present invention>

The present invention has a feature of providing a method for producing a lysophospholipid-containing composition, in which by subjecting a phospholipid to enzymolysis by phospholipase A in a polyhydric alcohol-based mixture containing a phospholipid, a fat and oil, a polyhydric alcohol, and a small amount of pure water as raw materials, and controlling the mixing ratio of the raw materials of the mixture such that the obtained composition has self-emulsifying properties, enzymolysis of the phospholipid by the phospholipase A can proceed without using an organic solvent accompanied by removal, and the obtained lysophospholipid-containing composition has self-emulsifying properties and is easily used for various applications.

### <Method for producing lysophospholipid-containing composition>

The method for producing a lysophospholipid-containing composition according to the embodiment of the present invention (hereinafter, also simply referred to as a "production method") includes a mixing step of uniformly mixing raw materials containing a phospholipid, a fat and oil, a polyhydric alcohol, and pure water with phospholipase A to initiate an enzymolysis of the phospholipid by phospholipase A, a phospholipid enzymolysis step of subjecting the phospholipid to the enzymolysis by phospholipase A such that a decomposition ratio of the phospholipid in a mixture obtained in the mixing step is 20% by mass or more, and an enzyme inactivation step of inactivating the phospholipase A in an enzymolysis product obtained in the phospholipid enzymolysis step.

### <Lysophospholipid-containing composition>

The lysophospholipid-containing composition obtained by the production method according to the embodiment of the present invention is a composition containing a lysophospholipid obtained by decomposing an ester bond between a glycero backbone and a fatty acid of a phospholipid with phospholipase A, using a phospholipid, a fat and oil, a polyhydric alcohol, and pure water as main raw materials, and having self-emulsifying properties described later. In addition, the lysophospholipid-containing composition obtained by the production method according to the embodiment of the present invention may contain other raw materials such as a food raw material, a pharmaceutical raw material, and a cosmetic raw material, in addition to the raw materials and those derived from the raw materials, as long as the composition has self-emulsifying properties described later. Furthermore, since the phospholipase A is subjected to an inactivation treatment, the lysophospholipid-containing composition obtained by the production method according to the embodiment of the present invention may be used as it is or in combination with other raw materials, and the combination thereof can be used for various applications such as food, pharmaceuticals, cosmetics, and additives for these applications.

### <Phospholipase A>

Phospholipase A, which is a phospholipid decomposition enzyme used in the production method according to the embodiment of the present invention, is an enzyme that decomposes an ester bond between a glycerol backbone and a fatty acid of a phospholipid to produce a lysophospholipid. Examples of the phospholipase A include phospholipase A1 and phospholipase A2. Phospholipase A is commercially available in a form derived from a microorganism, a form derived from an animal, and the like, but a form derived from a microorganism is preferable from the viewpoints of ease of enzyme inactivation treatment and CO₂ reduction in SDGs.

### <Amount of phospholipase A used>

The amount of phospholipase A used in the production method according to the embodiment of the present invention is not particularly limited as long as the decomposition ratio of the phospholipid subjected to enzymolysis by the phospholipase A is 20% or more. Although depending on the enzyme activity or the type of the phospholipase A to be used, the origin of the enzyme, the content of the phospholipid, or the like, as the amount of the phospholipase A used, specifically, the lower limit amount is, for example, preferably 0.001% or more, 0.005% or more, or 0.01% or more with respect to the total amount of the raw materials and the phospholipase A used in the mixing step, in consideration of the economic efficiency and the complexity of the inactivation step. The upper limit amount thereof is preferably 1.0% or less, 0.8% or less, or 0.5% or less.

### <Phospholipid and lysophospholipid>

The phospholipid used in the production method according to the embodiment of the present invention is a lipid involving phosphorus having two ester bonds between a glycerol backbone and a fatty acid. Specific examples thereof include phosphatidylcholine (hereinafter, abbreviated as "PC"), phosphatidylethanolamine (hereinafter, abbreviated as "PE"), phosphatidylinositol (hereinafter, abbreviated as "PI"), phosphatidylserine (hereinafter, abbreviated as "PS"), phosphatidic acid (hereinafter, abbreviated as "PA"), and the like.

On the other hand, the lysophospholipid is a lipid involving phosphorus having one ester bond between a glycerol backbone and a fatty acid. Specific examples thereof include lysophosphatidylcholine (hereinafter, abbreviated as "LPC"), lysophosphatidylethanolamine (hereinafter, abbreviated as "LPE"), lysophosphatidylinositol (hereinafter, abbreviated as "LPI"), lysophosphatidylserine (hereinafter, abbreviated as "LPS"), lysophosphatidic acid (hereinafter, abbreviated as "LPA"), and the like.

As the phospholipid used in the production method according to the embodiment of the present invention, one or two or more of the phospholipids can be used. In addition, in the present invention, an egg yolk phospholipid derived from egg yolk containing the one or two or more of phospholipids, a plant phospholipid derived from a plant such as soybean, sunflower, rapeseed, or rice, and further lecithin containing a fat and oil and the like such as triacylglycerol derived from each origin can also be used as a raw material.

In a case where lecithin containing a fat and oil and the like is used, the phospholipid moiety in the lecithin is the phospholipid of the present invention, and the fat and oil in the lecithin corresponds to the fat and oil described later.

### <Decomposition ratio of phospholipid>

In the production method according to the embodiment of the present invention, the decomposition ratio of the phospholipid by phospholipase A is 20% or more, preferably 25% or more, 30% or more, 35% or more, or 40% or more. In a case where the decomposition ratio is lower than the value, it is difficult to obtain a composition having self-emulsifying properties, which is the effect of the present invention, and it is not preferable.

### <Preparation of analytical sample for calculating decomposition ratio of phospholipid>

Regarding the decomposition ratio of the phospholipid specified in the present invention, a method of preparing a sample for analysis for calculating the decomposition ratio will be described below.
(1) 1.0 g of the lysophospholipid-containing composition obtained by the production method according to the embodiment of the present invention is separately collected into a 10 mL centrifuge tube with a screw cap, 2.0 mL of ion exchange water is added thereto, and the mixture is sufficiently stirred.
(2) 2.0 mL of methanol and 2.0 mL of chloroform are further sequentially added thereto, and the mixture is sufficiently stirred after each addition.
(3) The mixed solution in the test tube with a screw-cap is subjected to centrifugal separation at 3,000 rpm × 10 minutes to be separated into two layers.
(4) The chloroform-methanol layer as a lower layer separated into two layers is filtered through a membrane filter (PTFE type) having a pore diameter of 0.45 µm to prepare a sample for high performance liquid chromatography (HPLC).

### <High performance liquid chromatography (HPLC) analysis method for calculating decomposition ratio of phospholipid>

Next, the HPLC analysis conditions for calculating the decomposition ratio of the phospholipid are shown below. It is noted that conditions not specified in the following analysis conditions can be optionally set.
[Detector] Evaporative light scattering detector (ELSD): manufactured by Waters Corporation
[Column] Alltima HP Silica 5 µm, 250 mm × 4.6 mm
[Mobile phase A] Methanol:ion exchange water:acetic acid:triethylamine = 85:15:0.45:0.05 (volume ratio)
[Mobile phase B] Hexane:2-propanol:mobile phase A = 20:48:32 (volume ratio)

**[Table 1]**

| Gradient | | |
|---|---|---|
| Time (min) | Mobile phase A (%) | Mobile phase B (%) |
| 0 | 10 | 90 |
| 20 | 30 | 70 |
| 35 | 95 | 5 |
| 36 | 10 | 90 |
| 41 | 10 | 90 %: % by volume |

### <Method for calculating decomposition ratio of phospholipid>

The method for calculating the decomposition ratio of the phospholipid specified in the present invention is calculated by the following formula using the HPLC peak area of the following component after decomposition. Decomposition ratio (%) of phospholipid = [peak area of lysophospholipid/(peak area of phospholipid + peak area of lysophospholipid)] × 100

It is noted that in a case where the raw materials at the beginning of decomposition contains a lysophospholipid which is a component generated after decomposition, a value obtained by subtracting the content at the raw materials stage from the decomposition ratio of the phospholipid is defined as a decomposition ratio of the phospholipid.

In addition, in a case where a part or all of the phospholipid as raw materials contains PC, the decomposition ratio into the lysophospholipid and the decomposition ratio into LPC are substantially the same, and thus, in the present invention, the following formula is used. Decomposition ratio (%) of phospholipid = [peak area of LPC/(peak area of PC + peak area of LPC)] × 100

Since the peak area is proportional to the mass, the decomposition ratio (%) of the phospholipid calculated by the above formula is synonymous with % by mass.

Next, the fat and oil, polyhydric alcohol, and pure water, which are essential raw materials other than the phospholipid used in the production method according to the embodiment of the present invention, and other raw materials will be described.

### <Fat and oil>

Specific examples of the fat and oil used in the production method according to the embodiment of the present invention are triacylglycerol, diacylglycerol, and the like. As long as the effects of the present invention are not impaired, a fat and oil partially containing other neutral lipids, for example, free fatty acids, cholesterol, cholestanol, phytosterols, phytostanols, or the like can also be used. The origin of the fat and oil is not particularly limited, and examples thereof include a plant-derived fat and oil, an animal-derived fat and oil, an algae-derived fat and oil, a microorganism-derived fat and oil, and the like. From the viewpoint of SDGs, the fat and oil derived from plants, algae, or microorganisms other than animals is preferable, and the fat and oil derived from plants is more preferable. In addition, the fat and oil used in the production method according to the embodiment of the present invention is preferably a liquid fat and oil that maintains a liquid state even at room temperature (25°C), and more preferably a liquid fat and oil that maintains a liquid state even at refrigeration temperature (4°C). In the present invention, one or two or more of these can be used as the fat and oil.

It is noted that the fat and oil contained in the above-described lecithin is also included in the fat and oil of the present invention.

### <Polyhydric alcohol>

The polyhydric alcohol used in the production method according to the embodiment of the present invention is not particularly limited as long as it is an organic compound having two or more alcoholic hydroxy groups in one molecule, which is used as raw materials in the fields of food, pharmaceuticals, cosmetics, or the like. Specific examples of the polyhydric alcohol include hydrogenated starch syrup, sorbitol, xylitol, maltitol, glycerin, 1,3-butylene glycol, and the like. In the present invention, one or two or more of these can be used as the polyhydric alcohol. As the hydrogenated starch syrup, sorbitol, and the like, products containing 30% water are available on the market. In a case of such a product containing water, a portion excluding the water is the polyhydric alcohol of the present invention, and the water corresponds to the pure water described later.

### <Pure water>

The pure water used in the production method according to the embodiment of the present invention is not particularly limited as long as it is pure water used as raw materials in the fields of food, pharmaceuticals, cosmetics, or the like. Specific examples thereof include purified water, ion exchange water, distilled water, distilled water for injection, tap water, and the like. In the present invention, one or two or more of these can be used as the pure water.

The water contained in the polyhydric alcohol described above is also included in the pure water of the present invention, and the water contained in the aqueous raw materials that can be used in the production of the present invention, for example, the water contained in the starch syrup is also included in the pure water of the present invention.

### <Other raw materials>

The raw materials used in the production method according to the embodiment of the present invention include a phospholipid, a fat and oil, polyhydric alcohol, and pure water as essential raw materials. However, as long as the effects of the present invention are not impaired, other raw materials can be used in addition to these essential raw materials. Examples of the other raw materials include a pH adjuster, a chelating agent, an antioxidant, a flavoring raw material, a thickener, various vitamins, and the like.

Next, the amounts of the phospholipid, the fat and oil, the polyhydric alcohol, the pure water, and the phospholipase A used, which are essential raw materials used in the production method according to the embodiment of the present invention, will be described in detail. It is noted that the content of these essential raw materials used in the raw materials is synonymous with the amount used.

### <Total amount of essential raw materials and phospholipase A used>

The total amount of the phospholipid, the fat and oil, the polyhydric alcohol, the pure water, and the phospholipase A used in the mixing step is 90% or more, and preferably 95% or more or 98% or more with respect to the total amount of the raw materials and the phospholipase A used.

In a case where the total amount is less than the above-described amount, it may be difficult to homogenize the entire raw materials and phospholipase A, and the enzymolysis of the phospholipid by the phospholipase A may not proceed sufficiently.

### <Content of polyhydric alcohol in raw materials with respect to content of pure water in raw material>

In the production method according to the embodiment of the present invention, the content of the polyhydric alcohol in the raw materials is 0.4 to 3.0 times with respect to the content of the pure water in the raw material. In addition, since a high decomposition ratio of the phospholipids is easily obtained, the lower limit value is preferably 0.5 times or more, and the upper limit value is preferably 2.7 times or less.

In a case where the content of the polyhydric alcohol with respect to the content of the pure water is outside the above-described range, the entire mixture is separated after a while after the mixing is stopped even in a case of mixing the whole. Therefore, it is difficult to proceed with the enzymolysis of the phospholipid by phospholipase A while maintaining the entire mixture in a uniform state.

### <Content of phospholipid in raw materials with respect to total amount of phospholipid and fat and oil in raw material>

In the production method according to the embodiment of the present invention, the content of the phospholipid in the raw materials is 0.07 to 0.5 times with respect to the total amount of the phospholipid and the fat and oil in the raw material. In addition, since it is possible to produce the phospholipid in a state where the content of the phospholipid in the mixture obtained in the mixing step is relatively high, and it is easy to obtain a high decomposition ratio of the phospholipid, the lower limit value is preferably 0.08 times or more and 0.1 times or more, and the upper limit value is preferably 0.45 times or less and 0.4 times or less.

In a case where the content of the phospholipid with respect to the total amount of the phospholipid and the fat and oil is less than the lower limit value, the entire mixture is separated after a while after the mixing is stopped even in a case of mixing the whole. Therefore, it is difficult to proceed with the enzymolysis of the phospholipid by phospholipase A while maintaining the entire mixture in a uniform state. On the other hand, in a case where the value is more than the upper limit value, the enzymolysis of the phospholipid by phospholipase A does not proceed sufficiently.

### <Total amount of polyhydric alcohol and pure water in raw materials with respect to total amount of phospholipid and fat and oil in raw material>

In the production method according to the embodiment of the present invention, the total amount of the polyhydric alcohol and the pure water in the raw materials is 0.25 to 4.0 times with respect to the total amount of the phospholipid and the fat and oil in the raw material. In addition, since a high decomposition ratio of phospholipids is easily obtained, the lower limit value is preferably 0.3 times or more, 0.5 times or more, or 1.0 times or more, and the upper limit value is preferably 3.5 times or less.

In a case where the total amount of the polyhydric alcohol and the pure water with respect to the total amount of the phospholipid and the fat and oil is outside the above-described range, the entire mixture is separated after a while after the mixing is stopped even in a case of mixing the whole. Therefore, it is difficult to proceed with the enzymolysis of the phospholipid by phospholipase A while maintaining the entire mixture in a uniform state.

Although the conditions relating to the amount of the essential raw materials and phospholipase A used (the content in the raw materials and the like) have been described above, by satisfying the above-described conditions, in the mixture after the mixing step and at the beginning of the decomposition of the phospholipid enzymolysis step, since the entire mixture has a uniformity and an appropriate fluidity that facilitate enzymolysis of the entire mixture, the enzymolysis of the phospholipid by phospholipase A proceeds sufficiently, and since the mixture contains an appropriate phospholipid, the productivity of the obtained lysophospholipid is not deteriorated.

### <Self-emulsifying properties of lysophospholipid-containing composition>

The lysophospholipid-containing composition obtained by the production method according to the embodiment of the present invention has a characteristic that a value calculated by a L2 value - a L1 value is positive, preferably +5 or more or +10 or more, in a case where a brightness value (L1 value) of the composition is compared with a brightness value (L2 value) of an oil-in-water emulsion obtained by dispersing the composition in a 100 times by mass of pure water.

By controlling the blending ratio of raw materials and the like such that the obtained lysophospholipid-containing composition has the above-described characteristics, it is possible to sufficiently proceed with enzymolysis of the phospholipid by phospholipase A, and since the obtained composition has self-emulsifying properties, it is easy to use the composition for various applications of an emulsified system.

The fact that the brightness value, which is an indicator of brightness, of the composition dispersed in pure water is the same or higher than that of the obtained lysophospholipid-containing composition itself means that the turbidity has increased because of the dispersion in water.

In general, in a case where the oil-in-water emulsion is dispersed in pure water, the proportion of the oil phase constituting the emulsified particles in the oil-in-water emulsion is relatively reduced. Therefore, the degree of turbidity is reduced and the brightness value is also reduced.

On the other hand, it is presumed that the reason why the brightness value of the lysophospholipid-containing composition obtained by the production method according to the embodiment of the present invention dispersed in pure water is equal to or higher than that of the composition itself is that the dispersion state of the lipid components such as the lysophospholipid, the fat and oil, and the like in the composition is a state of being dispersed or dissolved in the polyhydric alcohol-based solvent and is different from that of the oil-in-water emulsion, and the self-emulsifying properties that enable the generation of new fine emulsified particles in pure water only by dispersing the composition in pure water are exhibited.

In the present invention, the brightness value (L value) can be measured using a color difference meter (trade name "Color Meter ZE-2000", manufactured by NIPPON DENSHOKU INDUSTRIES Co., Ltd.).

### Next, each step will be described.

### <Mixing step>

In the production method according to the embodiment of the present invention, in the mixing step, raw materials containing a phospholipid, a fat and oil, a polyhydric alcohol, and pure water, and phospholipase A are uniformly mixed. The state of being uniformly mixed is a state in which the raw materials and phospholipase A that are not dissolved and not dispersed are not observed with the naked eye, and even in a case where the mixing is stopped, the mixed state is maintained for a while (about 3 minutes). For example, a state such that after a while after the mixing is stopped, the entire mixture is separated, or the undispersed substance of the phospholipid is observed is not a state of being uniformly mixed.

### <Phospholipid enzymolysis step>

The phospholipid is subjected to enzymolysis by phospholipase A in the phospholipid enzymolysis step of the present invention such that the decomposition ratio of the phospholipid is 20% or more, preferably 25% or more, 30% or more, 35% or more, or 40% or more.

By adjusting the amounts of the essential raw materials and phospholipase A used to satisfy the above-described conditions, the enzymolysis proceeds smoothly.

In addition, since the enzymolysis is initiated at the same time as the addition of phospholipase A in the presence of the phospholipid, the mixing step and the phospholipid enzymolysis step proceed at the same time.

### <Enzyme inactivation step>

In the present invention, the phospholipase A is inactivated after the phospholipid is subjected to the enzymolysis to a desired decomposition ratio in the phospholipid enzymolysis step, and the decomposition reaction by the enzyme is stopped.

The method of inactivating phospholipase A is not particularly limited, and for example, in the present invention, a method of optionally inactivating the enzyme, such as a method of subjecting the obtained enzymolysis product to heat treatment at a temperature at which the enzyme is inactivated (for example, 70°C or higher) or a method of adjusting the pH of the obtained enzymolysis product to 4.5 or lower with a pH adjuster to stop the enzymolysis, can be adopted.

### <Method for producing oil-in-water emulsion composition using lysophospholipid-containing composition>

In the lysophospholipid-containing composition obtained by the production method according to the embodiment of the present invention, the oil-in-water emulsion composition can be obtained by dispersing the composition in pure water. Therefore, the present invention also relates to a method for producing an oil-in-water emulsion composition, the method including a water dispersion step of dispersing the lysophospholipid-containing composition obtained by the production method according to the embodiment of the present invention in pure water. The amount of the pure water used in the water dispersion step is preferably 0.5 times or more, 0.8 times or more, 1.0 times or more, or 2.0 times or more with respect to the lysophospholipid-containing composition. In addition, the obtained oil-in-water emulsion composition can be used as food, a pharmaceutical product, a cosmetic product, and the like. For example, in a case where the lysophospholipid-containing composition is dispersed in a mixed solution obtained by diluting soy sauce and vinegar with pure water, an emulsified liquid seasoning (oil-in-water emulsion composition) having a soy sauce flavor is obtained. It is noted that the amount of the pure water used is a total amount of the water of each of the soy sauce and the vinegar and the pure water used for dilution, in a case where the mixed solution is described as an example.

### <Method for producing mixed composition using lysophospholipid-containing composition>

As long as in the present invention, the self-emulsifying properties is exhibited, that is, the value calculated by a L2 value - a L1 value is positive, preferably +5 or more or +10 or more, one or two or more of a fat and oil, a lipid-soluble substance, a polyhydric alcohol, pure water, and a water-soluble substance can be added and mixed with the lysophospholipid-containing composition obtained by the production method according to the embodiment of the present invention. Therefore, the present invention also relates to a method for producing a mixed composition, the method including a mixing step of mixing one or two or more of a fat and oil, a lipid-soluble substance, a polyhydric alcohol, pure water, and a water-soluble substance with the lysophospholipid-containing composition obtained by the production method according to the embodiment of the present invention, in which a value calculated by a L2 value - a L1 value is positive in the mixed composition obtained by the mixing step.
The L1 value: a brightness value of the mixed composition
The L2 value: a brightness value of an oil-in-water emulsion obtained by dispersing the mixed composition in 100 times by mass of pure water

For example, for various purposes such as improvement of ease of use or preservation properties of the lysophospholipid-containing composition, and improvement of preservation stability of raw materials to be added and mixed, in the present invention, one or two or more of a fat and oil, a lipid-soluble substance, a polyhydric alcohol, pure water, and a water-soluble substance can be added and mixed with the lysophospholipid-containing composition.

The lipid-soluble substance to be added and mixed is not particularly limited as long as it is a substance that can be dissolved in a fat and oil or is dissolved in a fat and oil, and examples thereof include various lipid-soluble vitamins, spices, lipid-soluble pharmaceutical raw materials, lipid-soluble cosmetic raw materials, fragrances, and the like. In addition, the water-soluble substance is not particularly limited as long as it is a substance that can be dissolved in pure water or is dissolved in pure water, and examples thereof include liquid seasonings such as soy sauce and vinegar, water-soluble seasonings such as water-soluble vitamins, table salt, and sugar, various preservatives, water-soluble pharmaceutical raw materials, water-soluble cosmetic raw materials, and the like.

### <Method for producing oil-in-water emulsion composition using mixed composition>

In the mixed composition obtained by the method for producing a mixed composition according to the embodiment of the present invention, similarly as in the above-described lysophospholipid-containing composition, an oil-in-water emulsion composition is obtained by dispersing the composition in pure water. Therefore, the present invention also relates to a method for producing an oil-in-water emulsion composition, the method including a water dispersion step of dispersing the mixed composition obtained by the method for producing a mixed composition of the present invention in pure water. The amount of the pure water used in the water dispersion step is preferably 0.5 times or more, 0.8 times or more, 1.0 times or more, or 2.0 times or more with respect to the mixed composition. In addition, the obtained oil-in-water emulsion composition can be used as food, a pharmaceutical product, a cosmetic product, and the like.

### Examples

Hereinafter, the present invention will be specifically described based on examples, comparative examples, and test examples. It is noted that the present invention is not limited thereto.

### [Test Example 1]

In Test Example 1, the influence of the proportion of the content of the polyhydric alcohol in the raw materials to the content of the pure water in the raw materials on the enzymolysis reaction was examined.

The raw materials and phospholipase A shown in Table 2 were prepared, and the rapeseed oil and sunflower lecithin as oily raw materials and the hydrogenated starch syrup and the pure water as aqueous raw materials were each uniformly mixed to prepare an oily mixture and an aqueous mixture. Next, the oily mixture and the aqueous mixture were uniformly mixed with each other, and heated to 50°C, and then phospholipase A was added thereto to initiate enzymolysis of the phospholipid (mixing step). The phospholipid was subjected to the enzymolysis reaction for 24 hours (phospholipid enzymolysis step), heated to 80°C, and then treated at the same temperature for 30 minutes (enzyme inactivation step) for inactivating the enzyme.

After performing the enzyme inactivation step, the mixture was cooled to produce a lysophospholipid-containing composition according to the embodiment of the present invention. The results are shown in Table 2.

In the raw materials in Table 2, as sunflower lecithin, a trade name: Solec SF-10, manufactured by DuPont de Nemours, Inc., was used. The Solec SF-10 contains PC, PE, PI, PA, and the like as phospholipids, and the content of the phospholipid is about 60% as an acetone insoluble substance. Based on this, the calculation was performed by assuming that the content of the phospholipid in the sunflower lecithin in Table 2 was 60%, and the remaining 40% was the fat and oil. In addition, as phospholipase A2, a trade name: PLA2 NAGASE 10P/R, manufactured by Nagase ChemteX Corporation, was used. As other raw materials, commercially available raw materials were used.

It is noted that in Comparative Examples 1 and 2 in Table 2, the entire mixture was separated after a while from the mixing step, and thus the production was stopped. In the same manner as in the following test examples, in a case where the entire mixture was separated after the mixing step, the production was stopped. In the following test examples and examples, the same raw materials as in Test Example 1 were used and the production was performed by the same method. In addition, since PC was contained as a phospholipid in a part of the sunflower lecithin used, the decomposition ratio of the phospholipid was calculated from the above-described Formula 2 in the subsequent test examples and examples including Test Example 1.

**[Table 2]**

| | Comparative Example 1 | Example 1 | Example 2 | Example 3 | Example 4 | Example 5 | Example 6 | Comparative Example 2 |
|---|---|---|---|---|---|---|---|---|
| Rapeseed oil | 5.0 | 5.0 | 5.0 | 5.0 | 5.0 | 5.0 | 8.0 | 8.0 |
| Sunflower lecithin | 5.0 | 5.0 | 5.0 | 5.0 | 5.0 | 5.0 | 2.0 | 2.0 |
| Glycerin | 6.0 | 9.0 | 11.7 | 15.2 | 18.2 | 0.0 | 19.2 | 20.2 |
| Hydrogenated starch syrup (30% water) | 0.0 | 0.0 | 0.0 | 0.0 | 0.0 | 26.0 | 0.0 | 0.0 |
| Phospholipase A2 | 0.054 | 0.054 | 0.054 | 0.054 | 0.054 | 0.054 | 0.054 | 0.054 |
| Ion exchange water | 20.2 | 17.2 | 14.5 | 11.0 | 8.0 | 0.2 | 7.0 | 6.0 |
| Total (parts) | 36.25 | 36.25 | 36.25 | 36.25 | 36.25 | 36.25 | 36.25 | 36.25 |
| Phospholipid (parts) | 3.0 | 3.0 | 3.0 | 3.0 | 3.0 | 3.0 | 1.2 | 1.2 |
| Fat and oil (parts) | 7.0 | 7.0 | 7.0 | 7.0 | 7.0 | 7.0 | 8.8 | 8.8 |
| Polyhydric alcohol (parts) | 6.0 | 9.0 | 11.7 | 15.2 | 18.2 | 18.2 | 19.2 | 20.2 |
| Pure water (parts) | 20.2 | 17.2 | 14.5 | 11.0 | 8.0 | 8.0 | 7.0 | 6.0 |
| Polyhydric alcohol/pure water | 0.3 | 0.5 | 0.8 | 1.4 | 2.3 | 2.3 | 2.7 | 3.4 |
| Phospholipid/(phospholipid + fat and oil) | 0.3 | 0.3 | 0.3 | 0.3 | 0.3 | 0.3 | 0.12 | 0.12 |
| (Polyhydric alcohol + pure water)/(phospholipid + fat and oil) | 2.6 | 2.6 | 2.6 | 2.6 | 2.6 | 2.6 | 2.6 | 2.6 |
| State at beginning of enzymolysis | Separated | Uniform | Uniform | Uniform | Uniform | Uniform | Uniform | Separated |
| Decomposition ratio of phospholipid (%) | - | 100% | 98.1% | 96.3% | 89.6% | 91.7% | 100% | - |
| L2 - L1 | - | 10 or more | 10 or more | 10 or more | 10 or more | 10 or more | 10 or more | - |

From Table 2, in a case where the content of the polyhydric alcohol was in a range of 0.4 to 3.0 times, preferably in a range that the lower limit was 0.5 times or more and the upper limit was 2.7 times or less, with respect to the content of the pure water, the value calculated by a L2 value - a L1 value was +10 or more, which was positive, and the numerical value of the decomposition ratio of the phospholipid was also high. On the other hand, in a case of the content of the polyhydric alcohol was outside the above-described range, after the mixing step, the entire components were separated after a while and the enzymolysis reaction could not proceed in a uniform state.

In a case where the lysophospholipid-containing composition obtained in each of examples was dispersed in 0.5 times amount or more, 0.8 times amount or more, 1.0 times amount or more, and 2.0 times amount or more of pure water, an oil-in-water emulsion composition was obtained in all case.

### [Test Example 2]

In Test Example 2, the influence of the ratio of the content of the phospholipid in the raw materials to the total content of the phospholipid and the fat and oil in the raw materials on the enzymolysis reaction was examined.

The results are shown in Table 3.

**[Table 3]**

| | Comparative Example 3 | Comparative Example 4 | Example 7 | Example 8 | Example 9 | Example 10 | Example 5 | Example 11 | Comparative Example 5 |
|---|---|---|---|---|---|---|---|---|---|
| Rapeseed oil | 9.5 | 9 | 8.83 | 8.65 | 8.0 | 7.5 | 5.0 | 2.5 | 0.0 |
| Sunflower lecithin | 0.5 | 1.0 | 1.17 | 1.35 | 2.0 | 2.5 | 5.0 | 7.5 | 10.0 |
| Glycerin | 0.0 | 0.0 | 0.0 | 0.0 | 0.0 | 0.0 | 0.0 | 0.0 | 0.0 |
| Hydrogenated starch syrup (30% water) | 26.0 | 26.0 | 26.0 | 26.0 | 26.0 | 26.0 | 26.0 | 26.0 | 26.0 |
| Phospholipase A2 | 0.054 | 0.054 | 0.054 | 0.054 | 0.054 | 0.054 | 0.054 | 0.054 | 0.054 |
| Ion exchange water | 0.2 | 0.2 | 0.2 | 0.2 | 0.2 | 0.2 | 0.2 | 0.2 | 0.2 |
| Total (parts) | 36.25 | 36.25 | 36.25 | 36.25 | 36.25 | 36.25 | 36.25 | 36.25 | 36.25 |
| Phospholipid (parts) | 0.3 | 0.6 | 0.7 | 0.8 | 1.2 | 1.5 | 3.0 | 4.5 | 6.0 |
| Fat and oil (parts) | 9.7 | 9.4 | 9.3 | 9.2 | 8.8 | 8.5 | 7.0 | 5.5 | 4.0 |
| Polyhydric alcohol (parts) | 18.2 | 18.2 | 18.2 | 18.2 | 18.2 | 18.2 | 18.2 | 18.2 | 18.2 |
| Pure water (parts) | 8.0 | 8.0 | 8.0 | 8.0 | 8.0 | 8.0 | 8.0 | 8.0 | 8.0 |
| Polyhydric alcohol/pure water | 2.3 | 2.3 | 2.3 | 2.3 | 2.3 | 2.3 | 2.3 | 2.3 | 2.3 |
| Phospholipid/(phospholipid + fat and oil) | 0.03 | 0.06 | 0.07 | 0.08 | 0.12 | 0.15 | 0.3 | 0.45 | 0.6 |
| (Polyhydric alcohol + pure water)/(phospholipid + fat and oil) | 2.6 | 2.6 | 2.6 | 2.6 | 2.6 | 2.6 | 2.6 | 2.6 | 2.6 |
| State at beginning of enzymolysis | Separated | Separated | Uniform | Uniform | Uniform | Uniform | Uniform | Uniform | Uniform |
| Decomposition ratio of phospholipid (%) | - | - | 100% | 100% | 100% | 100% | 91.7% | 36.6% | 17.4% |
| L2 - L1 | - | - | 10 or more | 10 or more | 10 or more | 10 or more | 10 or more | 10 or more | - |

From Table 3, in a case where the content of the phospholipid was in a range of 0.07 to 0.5 times, preferably in a range that the lower limit was 0.08 times or more, or 0.1 times or more, and the upper limit was 0.45 times or less, or 0.4 times or less, with respect to the total content of the phospholipid and the fat and oil, the value calculated by a L2 value - a L1 value was +10 or more, which was positive, and the numerical value of the decomposition ratio of the phospholipid was also high. On the other hand, in a case of the content of the polyhydric alcohol was less than the above-described range, after the mixing step, the entire components were separated after a while and the enzymolysis reaction could not proceed in a uniform state. In a case where the value was more than the above-described range, the decomposition ratio of the phospholipid was less than 20%.

In a case where the lysophospholipid-containing composition obtained in each of examples was dispersed in 0.5 times amount or more, 0.8 times amount or more, 1.0 times amount or more, and 2.0 times amount or more of pure water, an oil-in-water emulsion composition was obtained in all case.

### [Test Example 3]

In Test Example 3, the influence of the ratio of the total content of the polyhydric alcohol and the pure water in the raw materials to the total content of the phospholipid and the fat and oil in the raw materials on the enzymolysis reaction was examined.

The results are shown in Table 4.

**[Table 4]**

| | Comparative Example 6 | Example 12 | Example 13 | Example 14 | Example 15 | Example 16 | Example 17 | Example 18 | Example 5 | Example 19 | Example 20 | Example 21 | Comparative Example 7 |
|---|---|---|---|---|---|---|---|---|---|---|---|---|---|
| Rapeseed oil | 8.0 | 7.0 | 6.0 | 5.0 | 5.0 | 5.0 | 5.0 | 5.0 | 5.0 | 4.5 | 4.5 | 4.0 | 3.0 |
| Sunflower lecithin | 8.0 | 7.0 | 6.0 | 5.0 | 5.0 | 5.0 | 5.0 | 5.0 | 5.0 | 4.5 | 4.5 | 4.0 | 3.0 |
| Glycerin | 2.3 | 2.5 | 2.5 | 0.0 | 3.5 | 0.0 | 7.0 | 0.0 | 0.0 | 0.0 | 18.2 | 18.2 | 18.2 |
| Hydrogenated starch syrup (30% water) | 0.0 | 0.0 | 0.0 | 5.0 | 0.0 | 10.0 | 0.0 | 15.0 | 26.0 | 26.0 | 0.0 | 0.0 | 0.0 |
| Phospholipase A2 | 0.054 | 0.054 | 0.054 | 0.054 | 0.054 | 0.054 | 0.054 | 0.054 | 0.054 | 0.054 | 0.054 | 0.054 | 0.054 |
| Ion exchange water | 1.0 | 1.1 | 1.1 | 0.2 | 1.5 | 0.2 | 3.2 | 0.2 | 0.2 | 0.2 | 8.0 | 8.0 | 8.0 |
| Total (parts) | 19.35 | 17.65 | 15.65 | 15.25 | 15.25 | 20.25 | 20.25 | 25.25 | 36.25 | 35.25 | 35.25 | 34.25 | 32.25 |
| Phospholipid (parts) | 4.8 | 4.2 | 3.6 | 3.0 | 3.0 | 3.0 | 3.0 | 3.0 | 3.0 | 2.7 | 2.7 | 2.4 | 1.8 |
| Fat and oil (parts) | 11.2 | 9.8 | 8.4 | 7.0 | 7.0 | 7.0 | 7.0 | 7.0 | 7.0 | 6.3 | 6.3 | 5.6 | 4.2 |
| Polyhydric alcohol (parts) | | 2.5 | 2.5 | 3.5 | 3.5 | 7.0 | 7.0 | 10.5 | 18.2 | 18.2 | 18.2 | 18.2 | 18.2 |
| Pure water (parts) | | 1.1 | 1.1 | 1.7 | 1.5 | 3.2 | 3.2 | 4.7 | 8.0 | 8.0 | 8.0 | 8.0 | 8.0 |
| Polyhydric alcohol/pure water | 2.3 | 2.3 | 2.3 | 2.1 | 2.3 | 2.2 | 2.2 | 2.2 | 2.3 | 2.3 | 2.3 | 2.3 | 2.3 |
| Phospholipid/(phospholipid + fat and oil) | 0.3 | 0.3 | 0.3 | 0.3 | 0.3 | 0.3 | 0.3 | 0.3 | 0.3 | 0.3 | 0.3 | 0.3 | 0.3 |
| (Polyhydric alcohol + pure water)/(phospholipid + fat and oil) | 0.21 | 0.26 | 0.3 | 0.5 | 0.5 | 1.0 | 1.0 | 1.5 | 2.6 | 2.9 | 2.9 | 3.3 | 4.4 |
| State at beginning of enzymolysis | Separated | Uniform | Uniform | Uniform | Uniform | Uniform | Uniform | Uniform | Uniform | Uniform | Uniform | Uniform | Separated |
| Decomposition ratio of phospholipid (%) | - | 27.4% | 44.1% | 27.7% | 64.0% | 34.2% | 90.0% | 55.7% | 91.7% | 67.7% | 81.4% | 81.0% | - |
| L2 - L1 | - | 10 or more | 10 or more | 10 or more | 10 or more | 10 or more | 10 or more | 10 or more | 10 or more | 10 or more | 10 or more | 10 or more | - |

From Table 4, in a case where the total content of the polyhydric alcohol and the pure water was in a range of 0.25 to 4.0 times, preferably in a range that the lower limit was 0.3 times or more, 0.5 times or more, or 1.0 times or more, and the upper limit was 3.5 times or less, with respect to the total content of the phospholipid and the fat and oil, the value calculated by a L2 value - a L1 value was +10 or more, which was positive, and the numerical value of the decomposition ratio of the phospholipid was also high depending on the type of the polyhydric alcohol used. On the other hand, in a case of the content of the polyhydric alcohol was outside the above-described range, after the mixing step, the entire components were separated after a while and the enzymolysis reaction could not proceed in a uniform state.

In a case where the lysophospholipid-containing composition obtained in each of examples was dispersed in 0.5 times amount or more, 0.8 times amount or more, 1.0 times amount or more, and 2.0 times amount or more of pure water, an oil-in-water emulsion composition was obtained in all case.

### [Example 22]

19.8 parts of hydrogenated starch syrup was mixed with 27.2 parts of the lysophospholipid-containing composition obtained in Example 9, and the mixture was uniformly mixed while gradually adding rapeseed oil, thereby obtaining 100 parts of a mixed composition. The obtained mixed composition was smoother and easier to use than the lysophospholipid-containing composition obtained in Example 9. In addition, the value calculated by a L2 value - a L1 value of the obtained mixed composition was +10 or more, which was positive.

In a case where the obtained mixed composition was dispersed in 0.5 times amount or more, 0.8 times amount or more, 1.0 times amount or more, and 2.0 times amount or more of pure water, an oil-in-water emulsion composition was obtained in all case.

## Claims

1. A method for producing a lysophospholipid-containing composition, in which a phospholipid is decomposed by phospholipase A, the method comprising:
a mixing step of uniformly mixing raw materials containing a phospholipid, a fat and oil, a polyhydric alcohol, and pure water, and phospholipase A to initiate enzymolysis of the phospholipid by the phospholipase A;
a phospholipid enzymolysis step of subjecting the phospholipid to the enzymolysis by the phospholipase A such that a decomposition ratio of the phospholipid in a mixture obtained in the mixing step is 20% by mass or more; and
an enzyme inactivation step of inactivating the phospholipase A in an enzymolysis product obtained in the phospholipid enzymolysis step,
wherein a value calculated by a L2 value - a L1 value is positive in the obtained lysophospholipid-containing composition,
the L1 value: a brightness value of the lysophospholipid-containing composition,
the L2 value: a brightness value of an oil-in-water emulsion obtained by dispersing the lysophospholipid-containing composition in 100 times by mass of pure water,
a total amount of the phospholipid, the fat and oil, the polyhydric alcohol, the pure water, and the phospholipase A used in the mixing step is 90% by mass or more with respect to a total amount of the raw materials and the phospholipase A used,
a content of the polyhydric alcohol in the raw materials is 0.4 to 3.0 times by mass with respect to a content of the pure water in the raw material,
a content of the phospholipid in the raw materials is 0.07 to 0.5 times by mass with respect to a total content of the phospholipid and the fat and oil in the raw material, and
a total content of the polyhydric alcohol and the pure water in the raw materials is 0.25 to 4.0 times by mass with respect to the total content of the phospholipid and the fat and oil in the raw material.

2. The method for producing a lysophospholipid-containing composition according to Claim 1,
wherein the value calculated by the L2 value - the L1 value is +5 or more.

3. A method for producing an oil-in-water emulsion composition, comprising:
a water dispersion step of dispersing the lysophospholipid-containing composition obtained by the method for producing a lysophospholipid-containing composition according to Claim 1 or 2 in pure water.

4. A method for producing a mixed composition, comprising:
a mixing step of mixing one or two or more of a fat and oil, a lipid-soluble substance, a polyhydric alcohol, pure water, and a water-soluble substance with the lysophospholipid-containing composition obtained by the method for producing a lysophospholipid-containing composition according to Claim 1 or 2,
wherein a value calculated by a L2 value - a L1 value is positive in the mixed composition obtained by the mixing step,
the L1 value: a brightness value of the mixed composition,
the L2 value: a brightness value of an oil-in-water emulsion obtained by dispersing the mixed composition in 100 times by mass of pure water.

5. A method for producing an oil-in-water emulsion composition, comprising:
a water dispersion step of dispersing the mixed composition obtained by the method for producing a mixed composition according to Claim 4 in pure water.
